# FASCICULE DE BREVET EUROPEEN

(11) **EP 1 872 773 B1**
(45) Date de publication et mention de la délivrance du brevet: **07.04.2010**
(21) Numéro de dépôt: 07109985.7
(22) Date de dépôt: 11.06.2007
(51) Int. Cl.: A61K 8/73, A61K 8/86, A61Q 5/00

(54) **Compositions cosmétiques contenant un amidon et un diester gras de PEG et leurs utilisations**
Stärke und PEG Diester Fettsäure enthaltende kosmetische Zusammensetzungen und ihre Verwendungen
Cosmetic compositions containing a starch and a PEG fatty diester, and uses thereof

(30) Priorité: 30.06.2006 FR 0652730
(43) Date de publication de la demande: 02.01.2008
(73) Titulaire: L'Oréal, 75008 Paris (FR)
(72) Inventeur: Mahe, Véronique, 78740, Vaux S/Seine (FR); Sturla, Jean-Michel, 92100, Boulogne (FR)
(74) Mandataire: Le Blainvaux Bellegarde, Françoise

(56) Documents cités:
- EP-A2- 1 051 967
- WO-A-03/084486
- DE-A1- 10 210 461
- FR-A1- 2 824 733

## Description

La présente invention concerne de nouvelles compositions cosmétiques comprenant dans un milieu cosmétiquement acceptable au moins un ester d'acide carboxylique particulier et au moins un amidon.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà proposé pour le traitement des matières kératiniques et en particulier des cheveux, des compositions cosmétiques contenant des polysaccharides épaississants tels que notamment l'amidon ou les celluloses.
De telles compositions présentent cependant des inconvénients tels que des problèmes de rinçabilité, des problèmes de stabilité à pH acide, des difficultés de répartition sur les matières kératiniques ainsi que des propriétés cosmétiques insuffisantes.

Les produits de soin capillaire, recherchés par les utilisateurs sont ceux que l'on peut doser et prendre aisément dans la main. Pour ce faire, ces produits doivent présenter une certaine consistance. En effet, un produit liquide est beaucoup plus difficile à doser et s'écoule facilement entre les doigts. En revanche, il s'applique mieux sur les cheveux et se répartit de façon homogène. Malheureusement, les produits épais sont généralement difficiles à étaler sur les cheveux et confèrent un soin non homogène à la chevelure. De plus, les produits, qu'ils soient épais ou liquides, peuvent ne pas conduire à la perception d'un effet traitant suffisant.

En résumé, il s'avère que les compositions cosmétiques actuelles ne donnent pas complètement satisfaction.

La demanderesse a maintenant découvert que l'association d'un amidon avec des esters carboxyliques particuliers permet de remédier à ces inconvénients.

Ainsi, à la suite d'importantes recherches menées sur la question, il a maintenant été trouvé par la Demanderesse que l'utilisation de compositions en particulier capillaires à base d'esters particuliers et d'amidon, permettait de limiter, voire supprimer, les problèmes évoqués ci-dessus.

Cette association apporte une texture fondante aux compositions cosmétiques. Les compositions se répartissent rapidement et facilement dans la chevelure tout en restant à la surface du cheveu. Par ailleurs, elles se rincent également très facilement

Les cheveux traités avec cette composition ont un toucher doux et sans résidus.
Les cheveux se démêlent facilement et sont lisses de la racine à la pointe, la tenue de la coiffure est améliorée.

Les compositions de l'invention ont un bon effet traitant qui ajouté aux bonnes propriétés de texture et de répartition du produit conduisent chez l'utilisateur à une sensation de soin profond.

On déjà proposé dans la demande FR2824733 d'associer des amidons à des esters gras liquides, cependant, les compositions obtenues ne présentent pas de bonnes propriétés de bonnes qualités d'usage. Notamment, la facilité de répartition du produit et l'effet traitant ressenti ne sont pas encore satisfaisants.

Par ailleurs, les compositions de l'invention appliquées sur la peau notamment sous forme de bain moussant ou de gel douche, apportent une amélioration de la douceur de la peau.

Ainsi, selon la présente invention, il est maintenant proposé de nouvelles compositions cosmétiques, comprenant, dans un milieu aqueux cosmétiquement acceptable,
- de 0,1 à 2% en poids par rapport au poids total de la composition d'au moins un amidon et
- de 0,1 à 1% en poids par rapport au poids total de la composition d'au moins un diester d'acide carboxylique ayant de 8 à 30 atomes de carbone et de polyéthylèneglycol, ledit diester ayant de 80 à 350 moles d'oxyde d'éthylène.

Un autre objet de l'invention concerne l'utilisation d'amidon dans, ou pour la fabrication d'une composition cosmétique comprenant un diester tel que défini ci-dessus.
Un autre objet de l'invention concerne l'utilisation pour le conditionnement des fibres kératiniques en particulier les cheveux d'une composition cosmétique telle que définie ci-dessus.

Un objet de l'invention concerne l'utilisation pour le traitement et/ou le conditionnement des fibres kératiniques en particulier les cheveux d'une composition cosmétique comprenant, dans un milieu cosmétiquement acceptable,
- de 0,1 à 2% en poids par rapport au poids total de la composition d'au moins un amidon et
- de 0,1 à 1% en poids par rapport au poids total de la composition d'au moins un diester d'acide carboxylique ayant de 8 à 30 atomes de carbone et de polyéthylèneglycol.

Les différents objets de l'invention vont maintenant être détaillés. L'ensemble des significations et définitions des composés utilisés dans la présente invention données ci-dessous sont valables pour l'ensemble des objets de l'invention.

Les amidons utilisables dans la présente invention sont plus particulièrement des macromolécules sous forme de polymères constitués de motifs élémentaires qui sont des unités anhydroglucose. Le nombre de ces motifs et leur assemblage permettent de distinguer l'amylose (polymère linéaire) et l'amylopectine (polymère ramifié). Les proportions relatives d'amylose et d'amylopectine, ainsi que leur degré de polymérisation, varient en fonction de l'origine botanique des amidons.

Les molécules d'amidons utilisés dans la présente invention peuvent avoir comme origine botanique les céréales ou encore les tubercules.
Ainsi, les amidons sont par exemple choisis parmi les amidons de maïs, de riz, de manioc, d'avoine, de tapioca, d'orge, de pomme de terre, de blé, de sorgho, de pois.
On peut également utiliser les hydrolysats des amidons cités ci-dessus. L'amidon est de préférence issu de la pomme de terre.

Les amidons se présentent généralement sous la forme d'une poudre blanche, insoluble dans l'eau froide, dont la taille des particules élémentaires va de 3 à 100 microns.

Les amidons utilisés dans la composition de l'invention peuvent être modifiés par une ou plusieurs des réactions suivantes : prégélatinisation, oxydation, réticulation, estérification, traitements thermiques.

De manière plus particulière, ces réactions peuvent être réalisées de la façon suivante :
- prégélatinisation en faisant éclater les granules d'amidon (par exemple séchage et cuisson dans un tambour sécheur) ;
- oxydation par des oxydants forts conduisant à l'introduction de groupes carboxyle dans la molécule d'amidon et à la dépolymèrisation de la molécule d'amidon (par exemple en traitant une solution aqueuse d'amidon par l'hypochlorite de sodium) ;
- réticulation par des agents fonctionnels capables de réagir avec les groupes hydroxyle des molécules d'amidon qui vont ainsi être liées entre elles (par exemple avec des groupes glyceryl et/ou phosphate) ;
- estérification en milieu alcalin pour le greffage de groupes fonctionnels, notamment acyl en en C1-C6 (acétyl), hydroxyalkylés en C1-C6 (hydroxyéthyl, hydroxypropyl), carboxyméthyl, octénylsuccinique.

On peut notamment obtenir par réticulation avec des composés phophorés des phosphates de monoamidon (du type Am-O-PO-(OX)₂), des phosphates de diamidon ( du type Am-O-PO-(OX)-O-Am) ou même de triamidon (du type Am-O-PO- (O-Am)₂) ou leurs mélanges.

X désigne notamment les métaux alcalins (par exemple sodium ou potassium), les métaux alcalinoterreux (par exemple calcium, magnésium), les sels d'ammoniaque, les sels d'amines comme ceux de la monoéthanolamine, la diéthanolamine, la triéthanolamine, l'amino-3 propanediol-1,2, les sels ammoniums issus des aminoacides basiques tels que la lysine, l'arginine, la sarcosine, l'ornithine, la citrulline.

Les composés phosphorés peuvent être par exemple du tripolyphosphate de sodium, de l'orthophosphate de sodium, de l'oxychlorure de phosphore ou du trimétaphosphate de sodium.

On utilisera préférentiellement des phosphates de diamidon ou des composés riches en phosphate de diamidon comme le produit proposé sous les références PREJEL VA-70-T AGGL (phosphate de diamidon de manioc hydroxypropylé gélatinisé) ou PREJEL TK1 (phosphate de diamidon de manioc gélatinisé) ou PREJEL 200 (phosphate de diamidon de manioc acétylé gélatinisé) par la Société AVEBE ou STRUCTURE ZEA de NATIONAL STARCH (phosphate de diamidon de maïs gélatinisé).

Selon l'invention, on peut aussi utiliser des amidons amphotères, ces amidons amphotères contiennent un ou plusieurs groupements anioniques et un ou plusieurs groupements cationiques. Les groupements anioniques et cationiques peuvent être liés au même site réactif de la molécule d'amidon ou à des sites réactifs différents; de préférence ils sont liés au même site réactif. Les groupements anioniques peuvent être de type carboxylique, phosphate ou sulfate et de préférence carboxylique. Les groupements cationiques peuvent être de type amine primaire, secondaire, tertiaire ou quaternaire.
Les amidons amphotères sont notamment choisis parmi les composés de formules suivantes : 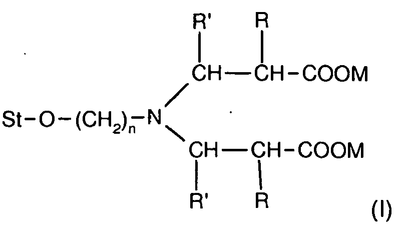 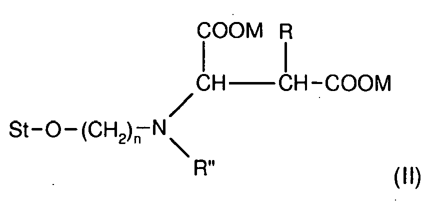 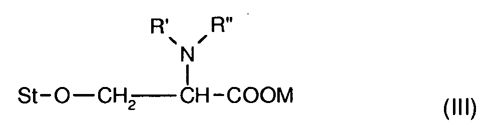 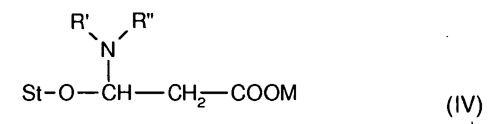 formules dans lesquelles :
St-O représente une molécule d'amidon,
R, identique ou différent, représente un atome d'hydrogène ou un radical méthyle,
R', identique ou différent, représente un atome d'hydrogène, un radical méthyle ou un groupement -COOH,
n est un entier égal à 2 ou 3,
M, identique ou différent, désigne un atome d'hydrogène, un métal alcalin ou alcalinoterreux tels que Na, K, Li, NH₄, un ammonium quaternaire ou une amine organique,
R" représente un atome d'hydrogène ou un radical alkyle ayant de 1 à 18 atomes de carbone.

Ces composés sont notamment décrits dans les brevets US 5,455,340 et US 4,017,460 qui sont inclus à titre de référence.

On utilise particulièrement les amidons de formules (I) ou (II). On utilise plus particulièrement les amidons modifiés par de l'acide 2-chloroéthyl aminodipropionique, c'est à dire les amidons de formule (I) ou (II) dans lesquelles R, R', R" et M représentent un atome d'hydrogène et n est égal à 2.

Le ou les amidons sont utilisés de préférence en une quantité allant de 0,1 à 1% en poids par rapport au poids total de la composition.

Les diesters d'acide carboxylique ayant de 8 à 30 atomes de carbone et de polyéthylèneglycol ont généralement de 80 à 350 moles d'oxyde d'éthylène (PEG).

Selon l'invention, les diesters d'acide carboxylique ayant de 8 à 30 atomes de carbone et de polyéthylèneglycol présentent généralement la formule suivante :

R1-CO(O-CH2-CH2)n - OOCR2 (I)

dans laquelle :
R1 désigne un groupement alkyle ou alcényle ayant de 8 à 30 atomes de carbone, linéaire ou ramifié, saturé ou insaturé,
R2 désigne un groupement alkyle ayant de 8 à 30 atomes de carbone, linéaire ou ramifié, saturé ou insaturé,
n est un nombre allant de 80 à 350.

De préférence R1 est un groupement alkyle linéaire ayant de 12 à 20 atomes de carbone. De préférence R2 est un groupement alkyle linéaire ayant de 12 à 20 atomes de carbone. De préférence n est un nombre compris entre 100 et 300.

De préférence, le rapport en poids de la partie hydrophile (-(O-CH2-CH2)nO) sur la partie hydrophobe (R1 et R2) est compris entre 8 et 1000.

On utilise de préférence un composé de formule (I) dans laquelle R1 et R2 désignent un groupement alkyle linéaire ayant de 12 à 20 atome de carbone, de préférence de 16 à 20 atomes de carbone et n est compris entre 100 et 300. On peut par exemple citer le distéarate de PEG 150 (n= 150) et le distéarate de PEG 250 (n = 250).

De tels composés sont notamment vendus sous la dénomination Emanon 3299R par la société KAO et sous la dénomination KESSCO PEG 6000 DS par la société AKZO.

Selon l'invention, le ou les diesters d'acide carboxylique et de PEG peuvent représenter de préférence de 0,1 % à 0,5 % en poids, du poids total de la composition finale.

Selon un mode de réalisation particulièrement préféré, les compositions selon l'invention comprennent en outre au moins un agent bénéfique pour les cheveux tels que notamment les silicones, les huiles végétales, animales, minérales ou de synthèse, les cires, les céramides, les pseudocéramides, les polymères cationiques, les tensioactifs en particulier cationiques, les esters gras différents de ceux de l'invention, les filtres solaires, les vitamines et leurs mélanges.

Les silicones utilisables conformément à l'invention sont en particulier des polyorganosiloxanes insolubles dans la composition et peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHONE POULENC, le décaméthylcyclopentasiloxane commercialisé sous le nom de "VOLATILE SILICONE 7158" par UNION CARBIDE, "SILBIONE 70045 V 5" par RHONE POULENC, ainsi que leurs mélanges.
   On peut également citer les cyclocopolymères du type diméthylsiloxanes/méthylakylsiloxane, tel que la "SILICONE VOLATILE FZ 3109" commercialisée par la société UNION CARBIDE, de structure chimique : 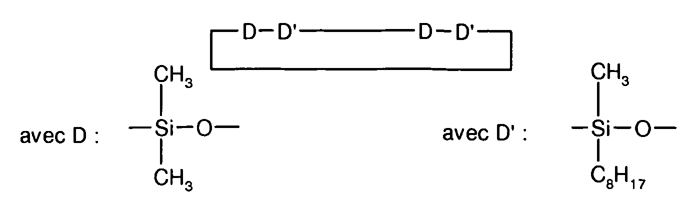 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHONE POULENC telles que par exemple l'huile 70 047 V 500 000 ;
- les huiles de la série MIRASIL commercialisées par la société RHONE POULENC ;
- les huiles de la série 200 de la société DOW CORNING telles que plus particulièrement la DC200 de viscosité 60 000 Cst ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHONE POULENC.

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 1.10⁻⁵ à 5.10⁻²m²/s à 25°C.

Parmi ces polyalkylarylsiloxanes on peut citer à titre d'exemple les produits commercialisés sous les dénominations suivantes :
- les huiles SILBIONE de la série 70 641 de RHONE POULENC ;
- les huiles des séries RHODORSIL 70 633 et 763 de RHONE POULENC ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane
- les gommes polydiméthylsiloxanes/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :
- les mélanges formés à partir d'un polydiméthylsiloxane hydroxylé en bout de chaîne (dénommé diméthiconol selon la nomenclature du dictionnaire CTFA) et d'un polydiméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités :
R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organo modifiées utilisables conformément à l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334 répondant à la formule (IX) : 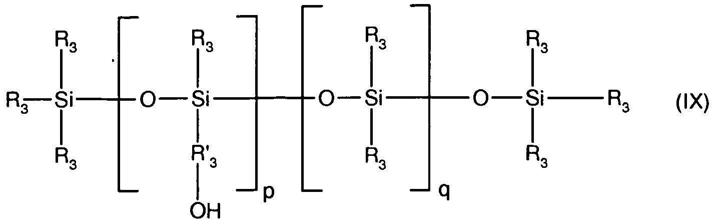 dans laquelle les radicaux R₃ identiques ou différents sont choisis parmi les radicaux méthyle et phényle ; au moins 60 % en mole des radicaux R₃ désignant méthyle ; le radical R'₃ est un chaînon alkylène divalent hydrocarboné en C₂-C₁₈ ; p est compris entre 1 et 30 inclus ; q est compris entre 1 et 150 inclus ;
- des groupements acyloxyalkyle tels que par exemple les polyorganosiloxanes décrits dans le brevet US-A-4957732 et répondant à la formule (X) : 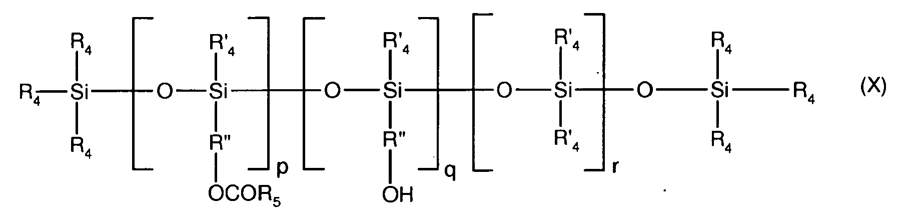 dans laquelle :
   R₄ désigne un groupement méthyle, phényle, -OCOR₅, hydroxyle, un seul des radicaux R₄ par atome de silicium pouvant être OH ;
   R'₄ désigne méthyle, phényle ; au moins 60 % en proportion molaire de l'ensemble des radicaux R₄ et R'₄ désignant méthyle ;
   R₅ désigne alkyle ou alcényle en C₈-C₂₀ ;
   R" désigne un radical alkylène hydrocarboné divalent, linéaire ou ramifié en C₂-C₁₈ ;
   r est compris entre 1 et 120 inclus ;
   p est compris entre 1 et 30 ;
   q est égal à 0 ou est inférieur à 0,5 p, p + q étant compris entre 1 et 30 ; les polyorganosiloxanes de formule (VI) peuvent contenir des groupements : 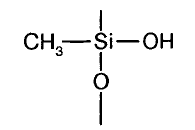 dans des proportions ne dépassant pas 15 % de la somme p + q + r.
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701 E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255".
- des groupements hydroxyacylamino, comme les polyorganosiloxanes décrits dans la demande EP 342 834. On peut citer par exemple le produit Q2-8413 de la société DOW CORNING.

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-412 704, EP-A-412 707, EP-A-640 105 et WO 95/00578, EP-A-582 152 et WO 93/23009 et les brevets US 4,693,935, US 4,728,571 et US 4,972,037. Ces polymères sont de préférence anioniques ou non ioniques.
De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule : 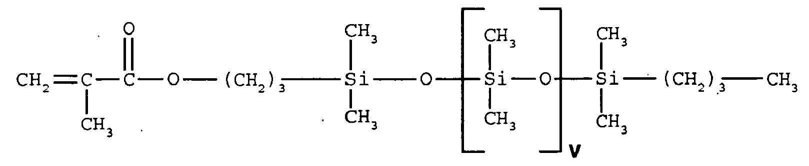
avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon l'invention, on peut également utiliser des polyuréthanes siliconés, notamment ceux décrits dans les demandes de brevet EP 0 751 162, EP 0 619 111.

Selon l'invention, toutes les silicones peuvent également être utilisées sous forme d'émulsions, de nanoémulsions ou de micrémulsions.

Les polyorganosiloxanes particulièrement préférés conformément à l'invention sont :
- les silicones non volatiles choisies dans la famille des polyalkylsiloxanes à groupements terminaux triméthylsilyle telles que les huiles ayant une viscosité comprise entre 0,2 et 2,5 m²/s à 25° C telles que les huiles de la séries DC200 de DOW CORNING en particulier celle de viscosité 60 000 Cst, des séries SILBIONE 70047 et 47 et plus particulièrement l'huile 70 047 V 500 000 commercialisées par la société RHONE POULENC, les polyalkylsiloxanes à groupements terminaux diméthylsilanol tels que les diméthiconol ou les polyalkylarylsiloxanes tels que l'huile SILBIONE 70641 V 200 commercialisée par la société RHONE POULENC ;
- la résine d'organopolysiloxane commercialisée sous la dénomination DOW CORNING 593 ;
- les polysiloxanes à groupements aminés tels que les amodiméthicones ou les triméthylsilylamodiméthicone ;

Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A- 0 337 354 et dans les demandes de brevets français FR-A- 2 270 846, 2 383 660, 2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Parmi tous les polymères cationiques susceptibles d'être utilisés dans le cadre de la présente invention, on préfère mettre en oeuvre les dérivés d'éther de cellulose quaternaires tels que les produits vendus sous la dénomination « JR 400 » par la Société UNION CARBIDE CORPORATION, les cyclopolymères cationiques, en particulier les homopolymères ou copolymères de chlorure de diméthyldiallylammonium, vendus sous les dénominations « MERQUAT 100 », « MERQUAT 550 » et « MERQUAT S » par la société CALGON, les polymères quaternaires de vinylpyrrolidone et de vinylimidazole, les homopolymères ou copolymères réticulés de sels de méthacryloyloxyalkyl(C₁-C₄) trialkyl(C₁-C₄)ammonium et leurs mélanges.

Selon l'invention, les agents bénéfiques additionnels peuvent représenter de 0,001 % à 20 % en poids, de préférence de 0,01 % à 10% en poids et plus particulièrement de 0,1 à 5% en poids par rapport au poids total de la composition finale.

Les compositions de l'invention contiennent en outre avantageusement au moins un agent tensioactif qui est généralement présent en une quantité comprise entre 0,01% et 50% en poids environ, de préférence entre 0,1% et 40% et encore plus préférentiellement entre 0,5% et 30%, par rapport au poids total de la composition.

Cet agent tensioactif peut être choisi parmi les agents tensioactifs anioniques, amphotères, non-ioniques, cationiques ou leurs mélanges.

Les tensioactifs convenant à la mise en oeuvre de la présente invention sont notamment les suivants :
(i) Tensioactif(s) anionique(s) :
   Leur nature ne revêt pas, dans le cadre de la présente invention, de caractère véritablement critique.
   Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.
   Parmi les tensioactifs anioniques, on préfère utiliser selon l'invention les sels d'alkylsulfates et d'alkyléthersufates et leurs mélanges.
(ii) Tensioactif(s) non ionique(s) :
   Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.
(iii) Tensioactif(s) amphotère(s):
   Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.
   Parmi les dérivés d'amines, on peut citer les produits commercialisés sous les dénomination MIRANOL, tels que décrits dans les brevets US-2 528 378 et US-2 781 354 et de structures :

   R₂ -CONHCH₂CH₂ -N(R₃)(R₄)(CH₂COO-) (2)

   dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
   et

   R₅-CONHCH₂CH₂-N(B)(C) (3)

   dans laquelle :
   B représente -CH₂CH₂OX', C représente -(CH₂)_{z} -Y', avec z = 1 ou 2,
   X' désigne le groupement -CH₂CH₂-COOH ou un atome d'hydrogène
   Y' désigne -COOH ou le radical -CH₂ - CHOH - SO3H
   R₅ désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.
   Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
   A titre d'exemple on peut citer le cocoamphodiacetate commercialisé sous la dénomination commerciale MIRANOL C2M concentré par la société RHONE POULENC.
(iv) Les tensioactifs cationiques peuvent être choisis parmi :
   A) les sels d'ammonium quaternaires de la formule générale (IV) suivante : 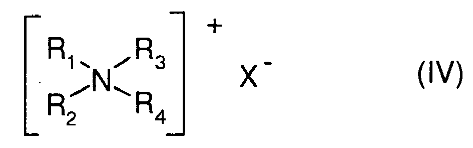 dans laquelle X est un anion choisi dans le groupe des halogénures (chlorure, bromure ou iodure) ou alkyl(C₂-C₆)sulfates plus particulièrement méthylsulfate, des phosphates, des alkyl-ou-alkylarylsulfonates, des anions dérivés d'acide organique tel que l'acétate ou le lactate.
      , et
      a) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
         R4 désigne un radical alkyle, linéaire ou ramifié, comportant de 16 à 30 atomes de carbone.
         De préférence le tensioactif cationique est un sel (par exemple chlorure) de béhényl triméthyl ammonium.
      b) les radicaux R1 et R2, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;
         R3 et R4, identiques ou différents, désignent un radical alkyle, linéaire ou ramifié, comportant de 12 à 30 atomes de carbone, ledit radical comprenant au moins une fonction ester ou amide.
         R3 et R4 sont notamment choisis parmi les radicaux alkyl(C₁₂-C₂₂)amido alkyle(C₂-C₆), alkyl(C₁₂-C₂₂)acétate ;
         De préférence le tensioactif cationique est un sel (par exemple chlorure) de stéaramidopropyl diméthyl (myristylacétate) ammonium.
   B) - les sels d'ammonium quaternaire de l'imidazolinium, comme par exemple celui de formule (V) suivante : 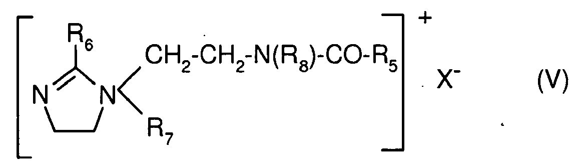 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,
   C) - les sels de diammonium quaternaire de formule (VI) : 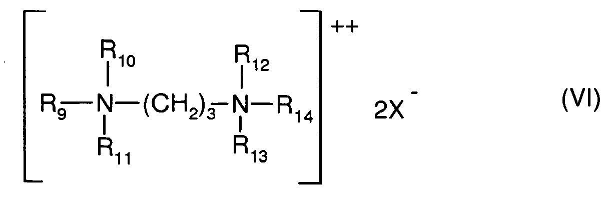 dans laquelle R₉ désigne un radical aliphatique comportant environ de 16 à 30 atomes de carbone, R₁₀, R₁₁, R₁₂, R₁₃ et R₁₄ , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
   D) - les sels d'ammonium quaternaire contenant au moins une fonction ester de formule (VII) suivante : 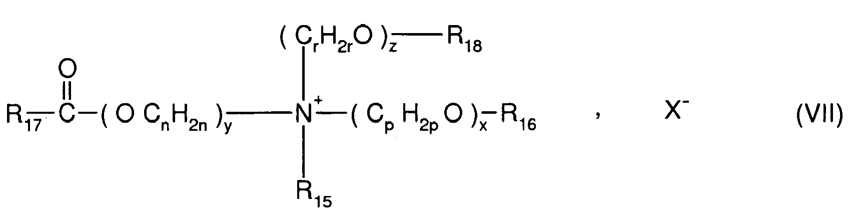 dans laquelle :
      - R₁₅ est choisi parmi les radicaux alkyles en C₁-C₆ et les radicaux hydroxyalkyles ou dihydroxyalkyles en C₁-C₆ ;
      - R₁₆ est choisi parmi :
         - le radical 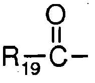
         - les radicaux R₂₀ hydrocarbonés en C₁-C₂₂ linéaires ou ramifiés, saturés ou insaturés,
         - l'atome d'hydrogène,
      - R₁₈ est choisi parmi :
         - le radical 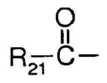
         - les radicaux R₂₂ hydrocarbonés en C₁-C₆ linéaires ou ramifiés, saturés ou insaturés,
         - l'atome d'hydrogène,
      - R₁₇, R₁₉ et R₂₁, identiques ou différents, sont choisis parmi les radicaux hydrocarbonés en C₇-C₂₁, linéaires ou ramifiés, saturés ou insaturés ;
      - n, p et r, identiques ou différents, sont des entiers valant de 2 à 6 ;
      - y est un entier valant de 1 à 10 ;
      - x et z, identiques ou différents, sont des entiers valant de 0 à 10 ;
      - X- est un anion simple ou complexe, organique ou inorganique ;
sous réserve que la somme x + y + z vaut de 1 à 15 , que lorsque x vaut 0 alors R₁₆ désigne R₂₀ et que lorsque z vaut 0 alors R₁₈ désigne R₂₂.

On utilise plus particulièrement les sels d'ammonium de formule (VII) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
   - le radical 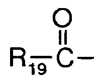
   - les radicaux méthyle, éthyle ou hydrocarbonés en C₁₄-C₂₂
   - l'atome d'hydrogène ;
- R₁₇, R₁₉ et R₂₁, identiques ou différents, sont choisis parmi les radicaux hydrocarbonés en C₇-C₂₁, linéaires ou ramifiés, saturés ou insaturés ;
- R₁₈ est choisi parmi :
   - le radical 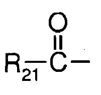
   - l'atome d'hydrogène ;

De tels composés sont par exemple commercialisés sous les dénominations DEHYQUART par la société HENKEL, STEPANQUAT par la société STEPAN, NOXAMIUM par la société CECA, REWOQUAT WE 18 par la société REWO-WITCO.

Parmi les sels d'ammonium quaternaire on préfère le chlorure de béhényltriméthylammonium, ou encore, le chlorure de stéaramidopropyldiméthyl (myristyl acétate) ammonium par exemple commercialisé sous la dénomination «CERAPHYL 70» par la société VAN DYK, le Quaternium-27 ou le Quaternium-83 commercialisés par la société WITCO, le méthosulfate de dicétéaroyléthyl hydroxyéthyl méthyl ammonium notamment commercialisé par la société COGNIS sous la dénomination DEHYQUART F75.

Le milieu aqueux cosmétiquement acceptable comprend uniquement de l'eau ou un mélange d'eau et d'un solvant cosmétiquement acceptable choisi parmi les alcools inférieurs en C₁-C₄, tels que l'éthanol, l'isopropanol, le tertio-butanol ou le n-butanol ; les polyols comme le propylèneglycol ; les éthers de polyols ; les alcanes en C₅-C₁₀ ; les cétones en C₃₋₄ comme l'acétone et la méthyléthylcétone ; les acétates d'alkyle en C₁-C₄ comme l'acétate de méthyle, l'acétate d'éthyle et l'acétate de butyle ; le diméthoxyéthane, le diéthoxyéthane ; et leurs mélanges.

La concentration en solvants cosmétiquement acceptables différents de l'eau (lorsqu'ils sont présents) va généralement de 0,1 à 40% en poids, de préférence de 0,5 à 20% en poids par rapport au poids total de la composition.

La composition comprend de préférence de 30 à 99% d'eau, en particulier de 50 à 95% d'eau et encore plus particulièrement de 70 à 95% en poids d'eau par rapport au poids total de la composition.

Le pH des compositions de l'invention est compris entre 4 et 8, de préférence entre 5 et 7.

La composition de l'invention peut également contenir au moins un additif choisi parmi les épaississants, les parfums, les agents nacrants, les conservateurs, les vitamines, les provitamines, les polymères anioniques ou non ioniques, les protéines, les hydrolysats de protéines, l'acide méthyl-18 eicosanoique, les hydroxyacides, les provitamines telles que le panthénol, et tout autre additif classiquement utilisé dans le domaine cosmétique qui n'affecte pas les propriétés des compositions selon l'invention.

L'homme de métier veillera à choisir les éventuels additifs et leur quantité de manière à ce qu'ils ne nuisent pas aux propriétés des compositions de la présente invention.

Ces additifs sont éventuellement présents dans la composition selon l'invention dans des proportions pouvant aller de 0,001 à 20% en poids par rapport au poids total de la composition. La quantité précise de chaque additif est déterminée facilement par l'homme du métier selon sa nature et sa fonction.

Les compositions conformes à l'invention peuvent être plus particulièrement utilisées pour le soin et/ou le traitement des matières kératiniques telles que les cheveux, la peau, les cils, les sourcils, les ongles, les lèvres, le cuir chevelu et plus particulièrement les cheveux.

Les compositions de l'invention peuvent se présenter sous forme d'après-shampooings à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Selon un mode de réalisation préféré de l'invention, la composition peut être utilisée comme après-shampoing.

Lorsque la composition se présente sous la forme d'un après-shampooing ou d'une composition de soin éventuellement à rincer, elle contient avantageusement au moins un tensioactif cationique, sa concentration est généralement comprise de 0,1 à 10% en poids et de préférence de 0,5 à 5% en poids par rapport au poids total de la composition. Elle peut également contenir un ou plusieurs tensioactifs amphotères ou non ioniques.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.

Ainsi, ce procédé selon l'invention permet le maintien de la coiffure, le traitement, le soin de la peau, des cheveux ou de toute autre matière kératinique et plus particulièrement les cheveux.

Les compositions selon l'invention peuvent également se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse et être utilisées pour la peau, les ongles, les cils, les lèvres et plus particulièrement les cheveux.

Les compositions peuvent être conditionnées sous diverses formes notamment dans des pots, des flacons, des tubes, des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits.
Dans les exemples, MA signifie matière active.

### EXEMPLES 1 et 2

On a réalisé deux après-shampooing conformes à l'invention de composition suivante :

| | 1 | 2 | A (comparatif) | B (comparatif) | C (comparatif) |
|---|---|---|---|---|---|
| Phosphate de diamidon de maïs prégélatinisé (STRUCTURE ZEA de NATIONAL STARCH) | 0,5 g | 0,2 g | - | | 2 g |
| Distéarate de PEG-150 (KESSCO PEG 6000 DS de AKZO) | 0,5 g | 0,3 g | - | 0,5 g | |
| Polyquaternium-37 (SALCARE SC 95 de CIBA GEIGY | 1 g MA | 1 g MA | 1 g MA | 1 g MA | 1 g MA |
| Copolymère SMDI / polyéthylène glycol à terminaisons alkyle (méthyl/C18) à 15% dans une matrice maltodextrine / eau (ACULYN 46 de ROHM HAAS) | 3 g MA | 3 g MA | 3 g MA | 3 g MA | 3 g MA |
| Alcool cétylstéarylique (50/50 en poids) | 8 g | 8 g | 8 g | 8 g | 8 g |
| Cétyl esters : mélange de myristate/palmitate/stéarate de myristyle/cétyle/stéaryle (MIRACETI de LASERSON) | 1,5 g | 1,5 g | 1,5 g | 1,5 g | 1,5 g |
| Cire de candelilla (Candellila wax SP 75 de STRAHL and PITSCH) | 2 g | 2 g | 2 g | 2 g | 2 g |
| Parfum, conservateur | qs | qs | qs | qs | qs |
| Eau qsp | 100 g | 100 g | 100 g | 100 g | 100 g |

Les compositions 1 et 2 ont une texture crémeuse et très fondante lors de l'application sur des cheveux humides, tout en apportant une présence sensorielle traitante sur les cheveux. Les compositions 1 et 2 se rincent facilement. Les cheveux sont doux et lisses.

Les compositions A, B et C sont crémeuses mais elles ne conduisent pas à la perception d'un effet traitant suffisant.

### EXEMPLE 3

On a réalisé un après-shampooing conforme à l'invention de composition suivante :

| | 3 | D (comparatif) | E (comparatif) | F (comparatif) |
|---|---|---|---|---|
| Phosphate de diamidon de maïs prégélatinisé (STRUCTURE ZEA de NATIONAL STARCH) | 0,2 g | - | | 0,5 g |
| Distéarate de PEG-150 (KESSCO PEG 6000 DS de AKZO) | 0,3 g | - | 0,5 g | |
| Stéarate de glycérol (TEGIN 6070 de DEGUSSA) | 1 g | 1 g | 1 g | 1 g |
| Chlorure de cétyl triméthyl ammonium (ARQUAD 16-25 LO d'AKZO NOBEL) | 0,75 gMA | 0,75 gMA | 0,75 gMA | 0,75 gMA |
| Quaternium-80 (ABILQUAT 3272 de DEGUSSA) | 0,3 g MA | 0,3 g MA | 0,3 g MA | 0,3 g MA |
| Méthosulfate de Dipalmitoyl éthyl hydroxyéthyl ammonium (DEHYQUART F75 de COGNIS) | 1,7 gMA | 1,7 gMA | 1,7 gMA | 1,7 gMA |
| Alcool cétylstéarylique (50/50 en poids) | 2,8 g | 2,8 g | 2,8 g | 2,8 g |
| Cétyl esters : mélange de myristate/palmitate/stéarate de myristyle/cétyle/stéaryle (MIRACETI de LASERSON) | 1,5 g | 1,5 g | 1,5 g | 1,5 g |
| Eau qsp | 100 g | 100 g | 100 g | 100 g |

La composition 3 a une texture crémeuse et très fondante lors de l'application sur des cheveux humides, tout en apportant une présence sensorielle traitante sur les cheveux. La composition 3 se rince facilement. Les cheveux sont doux et lisses.

Les compositions D, E et F sont crémeuses mais elles ne conduisent pas à la perception d'un effet traitant suffisant.

## Revendications

1. Composition cosmétique, **caractérisée par le fait qu'**elle comprend, dans un milieu cosmétiquement acceptable,
- de 0,1 à 2% en poids par rapport au poids total de la composition d'au moins un amidon et
- de 0,1 à 1% en poids par rapport au poids total de la composition d'au moins un diester d'acide carboxylique ayant de 8 à 30 atomes de carbone et de polyéthylèneglycol, ledit diester ayant de 80 à 350 moles d'oxyde d'éthylène.

2. Composition selon la revendication 1, **caractérisée par le fait que** les amidons sont choisis parmi les amidons de maïs, de riz, de manioc, d'avoine, de tapioca, d'orge, de pomme de terre, de blé, de sorgho, de pois.

3. Composition selon la revendication précédente, **caractérisée par le fait que** l'amidon est modifié par une ou plusieurs des réactions suivantes : prégélatinisation, oxydation, réticulation, estérification, traitements thermiques.

4. Composition selon l'une quelconque des revendications 1 à 3, **caractérisée par le fait que** l'amidon est choisi parmi les phosphates de mono amidon, les phosphates de diamidon, les phosphates de triamidon et leurs mélanges.

5. Composition selon l'une quelconque des revendications 1 à 4, **caractérisée par le fait que** l'amidon est choisi parmi les amidons amphotères.

6. Composition selon l'une quelconque des revendications 1 à 5, **caractérisée par le fait que** les diesters d'acide carboxylique ayant de 8 à 30 atomes de carbone et de polyéthylèneglycol présentent la formule suivante:
R1-CO(O-CH2-CH2)n - OOCR2 (I)
dans laquelle :
R1 désigne un groupement alkyle ou alcényle ayant de 8 à 30 atomes de carbone, linéaire ou ramifié, saturé ou insaturé,
R2 désigne un groupement alkyle ayant de 8 à 30 atomes de carbone, linéaire ou ramifié, saturé ou insaturé,
n est un nombre allant de 80 à 350.

7. Composition selon la revendication 6, **caractérisée par le fait que** R1 et R2 désignent un groupement alkyle linéaire ayant de 12 à 20 atome de carbone et n va de 100 à 300.

8. Composition selon l'une quelconque des revendications précédentes, **caractérisée en ce que** l'amidon est présent à une concentration allant de 0,1 % à 1 % en poids par rapport au poids total de la composition.

9. Composition selon l'une quelconque des revendications précédentes, **caractérisée en ce que** le diester d'acide carboxylique est présent à une concentration allant de 0,1 % à moins de 0,5 % en poids par rapport au poids total de la composition.

10. Composition selon l'une quelconque des revendications 1 à 9 **caractérisée par le fait qu'**elle comprend en outre au moins un agent bénéfique pour les cheveux choisi parmi les huiles végétales, animales, minérales ou de synthèse, les cires, les céramides, les pseudocéramides, lés silicones, les polymères cationiques, les tensioactifs, les esters gras différents des diester d'acide carboxylique ayant de 8 à 30 atomes de carbone et de polyéthylèneglycol, les filtres solaires, les vitamines.

11. Composition selon la revendication 10, **caractérisée en ce que** l'agent bénéfique pour les cheveux est présent à une concentration allant de 0,001 % à 20 % en poids par rapport au poids total de la composition, de préférence de 0,01 % à 10 % en poids.

12. Composition selon l'une quelconque des revendications précédentes, **caractérisée par le fait qu'**elle comprend en outre au moins un agent tensioactif choisi parmi les tensioactifs anioniques, non ioniques, amphotères, cationiques et leurs mélanges.

13. Composition selon la revendication précédente, **caractérisée par le fait qu'**elle comprend en outre au moins un agent tensioactif choisi parmi les tensioactifs cationiques et leurs mélanges.

14. Composition selon la revendication 13, **caractérisée par le fait que** le ou les agents tensioactifs cationiques sont présents à une concentration allant de 0,1 à 10% en poids et de préférence de 0,5 à 5% en poids par rapport au poids total de la composition.

15. Composition selon l'une quelconque des revendications précédentes, **caractérisée par le fait qu'**elle se présente sous forme d'après-shampooing, de composition pour la permanente, le défrisage, la coloration ou la décoloration des cheveux, de composition à rincer à appliquer entre les deux étapes d'une permanente ou d'un défrisage.

16. Utilisation d'une composition telle que définie dans l'une quelconque des revendications précédentes pour le soin des matières kératiniques en particulier les cheveux.

17. Utilisation d'une composition selon l'une quelconque des revendications 1 à 15, comme après-shampoing.

18. Utilisation d'une composition cosmétique, **caractérisée par le fait qu'**elle comprend, dans un milieu cosmétiquement acceptable,
- de 0,1 à 2% en poids par rapport au poids total de la composition d'au moins un amidon et
- de 0,1 à 1% en poids par rapport au poids total de la composition d'au moins un diester d'acide carboxylique ayant de 8 à 30 atomes de carbone et de polyéthylèneglycol.
pour le traitement et/ou le conditionnement des cheveux.

19. Utilisation selon la revendication 18, comme après-shampoing.

20. Procédé de traitement des cheveux, **caractérisé en ce qu'**il consiste à appliquer sur lesdits cheveux une composition cosmétique comprenant, dans un milieu cosmétiquement acceptable,
- de 0,1 à 2% en poids par rapport au poids total de la composition d'au moins un amidon et
- de 0,1 à 1 % en poids par rapport au poids total de la composition d'au moins un diester d'acide carboxylique ayant de 8 à 30 atomes de carbone et de polyéthylèneglycol.
, puis à effectuer éventuellement un rinçage à l'eau.

## Claims

1. Cosmetic composition, **characterized in that** it comprises, in a cosmetically acceptable medium:
- from 0.1% to 2% by weight, relative to the total weight of the composition, of at least one starch, and
- from 0.1% to 1% by weight, relative to the total weight of the composition, of at least one diester of a carboxylic acid containing from 8 to 30 carbon atoms and of polyethylene glycol, the said diester containing from 80 to 350 mol of ethylene oxide.

2. Composition according to Claim 1, **characterized in that** the starches are chosen from corn starch, rice starch, cassava starch, oat starch, tapioca starch, barley starch, potato starch, wheat starch, sorghum starch and pea starch.

3. Composition according to the preceding claim, **characterized in that** the starch is modified by one or more of the following reactions: pregelatinization, oxidation, crosslinking, esterification, heat treatments.

4. Composition according to any one of Claims 1 to 3, **characterized in that** the starch is chosen from monostarch phosphates, distarch phosphates and tristarch phosphates, and mixtures thereof.

5. Composition according to any one of Claims 1 to 4, **characterized in that** the starch is chosen from amphoteric starches.

6. Composition according to any one of Claims 1 to 5, **characterized in that** the diesters of a carboxylic acid containing from 8 to 30 carbon atoms and of polyethylene glycol have the following formula:
R₁-CO(O-CH₂-CH₂)n-OOCR₂ (I)
in which:
R₁ denotes a linear or branched, saturated or unsaturated alkyl or alkenyl group containing from 8 to 30 carbon atoms,
R₂ denotes a linear or branched, saturated or unsaturated alkyl group containing from 8 to 30 carbon atoms,
n is a number ranging from 80 to 350.

7. Composition according to Claim 6, **characterized in that** R₁ and R₂ denote a linear alkyl group containing from 12 to 20 carbon atoms and n ranges from 100 to 300.

8. Composition according to any one of the preceding claims, **characterized in that** the starch is present in a concentration ranging from 0.1% to 1% by weight relative to the total weight of the composition.

9. Composition according to any one of the preceding claims, **characterized in that** the carboxylic acid diester is present in a concentration ranging from 0.1% to less than 0.5% by weight relative to the total weight of the composition.

10. Composition according to any one of Claims 1 to 9, **characterized in that** it also comprises at least one agent that is beneficial for the hair, chosen from plant, animal, mineral or synthetic oils, waxes, ceramides, pseudoceramides, silicones, cationic polymers, surfactants, fatty esters other than diesters of a carboxylic acid containing from 8 to 30 carbon atoms and of polyethylene glycol, sunscreens and vitamins.

11. Composition according to Claim 10, **characterized in that** the agent that is beneficial for the hair is present in a concentration ranging from 0.001% to 20% by weight and preferably from 0.01% to 10% by weight relative to the total weight of the composition.

12. Composition according to any one of the preceding claims, **characterized in that** it also comprises at least one surfactant chosen from anionic, nonionic, amphoteric and cationic surfactants, and mixtures thereof.

13. Composition according to the preceding claim, **characterized in that** it also comprises at least one surfactant chosen from cationic surfactants, and mixtures thereof.

14. Composition according to Claim 13, **characterized in that** the cationic surfactant(s) is (are) present in a concentration ranging from 0.1% to 10% by weight and preferably from 0.5% to 5% by weight relative to the total weight of the composition.

15. Composition according to any one of the preceding claims, **characterized in that** it is in the form of a hair conditioner, a composition for permanent-waving, relaxing, dyeing or bleaching the hair, or a rinse-out composition to be applied between the two steps of a permanent-waving or hair-relaxing operation.

16. Use of a composition as defined in any one of the preceding claims, for caring for keratin materials, in particular the hair.

17. Use of a composition according to any one of Claims 1 to 15, as a hair conditioner.

18. Use of a cosmetic composition, **characterized in that** it comprises, in a cosmetically acceptable medium,
- from 0.1% to 2% by weight, relative to the total weight of the composition, of at least one starch, and
- from 0.1% to 1% by weight, relative to the total weight of the composition, of at least one diester of a carboxylic acid containing from 8 to 30 carbon atoms and of polyethylene glycol,
for treating and/or conditioning the hair.

19. Use according to Claim 18, as a hair conditioner.

20. Hair treatment process, **characterized in that** it consists in applying to the said hair a cosmetic composition comprising, in a cosmetically acceptable medium:
- from 0.1% to 2% by weight, relative to the total weight of the composition, of at least one starch, and
- from 0.1% to 1% by weight, relative to the total weight of the composition, of at least one diester of a carboxylic acid containing from 8 to 30 carbon atoms and of polyethylene glycol,
and then in optionally rinsing with water.

## Patentansprüche

1. Kosmetische Zusammensetzung, **dadurch gekennzeichnet, dass** sie in einem kosmetisch akzeptablen Medium enthält:
- 0,1 bis 2 Gew.-% mindestens einer Stärke, bezogen auf das Gesamtgewicht der Zusammensetzung, und
- 0,1 bis 1 Gew.-% mindestens eines Diesters einer Carbonsäure mit 8 bis 30 Kohlenstoffatomen und Polyethylenglycol, bezogen auf das Gesamtgewicht der Zusammensetzung, wobei der Diester 80 bis 350 mol Ethylenoxid aufweist.

2. Zusammensetzung nach Anspruch 1, **dadurch gekennzeichnet, dass** die Stärke unter den Stärken aus Mais, Reis, Maniok, Hafer, Tapioca, Gerste, Kartoffel, Weisen, Sorgho und Erbse ausgewählt sind.

3. Zusammensetzung nach dem vorhergehenden Anspruch, **dadurch gekennzeichnet, dass** die Stärke durch eine oder mehrere der folgenden Reaktionen modifiziert ist:
Vorverkleisterung, Oxidation, Vernetzung, Veresterung, thermische Behandlungen.

4. Zusammensetzung nach einem der Ansprüche 1 bis 3, **dadurch gekennzeichnet, dass** die Stärke unter den Monostärkephosphaten, Distärkephosphaten, Tristärkephosphaten und deren Gemischen ausgewählt ist.

5. Zusammensetzung nach einem der Ansprüche 1 bis 4, **dadurch gekennzeichnet, dass** die Stärke unter den amphoteren Stärken ausgewählt ist.

6. Zusammensetzung nach einem der Ansprüche 1 bis 5, **dadurch gekennzeichnet, dass** die Diester einer Carbonsäure mit 8 bis 30 Kohlenstoffatomen und Polyethylenglycol die folgende Formel aufweisen:
R1-CO(O-CH2-CH2)n - OOCR2 (I)
in der Formel:
R1 bedeutet eine geradkettige oder verzweigte, gesättigte öder ungesättigte Alkyl- oder Alkenylgruppe mit 8 bis 30 Kohlenstoffatomen,
R2 bedeutet eine geradkettige oder verzweigte, gesättigte oder ungesättigte Alkylgruppe mit 8 bis 30 Kohlenstoffatomen,
n ist eine Zahl im Bereich von 80 bis 350.

7. Zusammensetzung nach Anspruch 6, **dadurch gekennzeichnet, dass** die Gruppen R 1 und R2 eine geradkettige Alkylgruppe mit 12 bis 20 Kohlenstoffatomen bedeuten und n im Bereich von 100 bis 300 liegt.

8. Zusammensetzung nach einem der vorhergehenden Ansprüche, **dadurch gekennzeichnet, dass** die Stärke in einer Konzentration von 0,1 bis 1 Gew.-%, bezogen auf das Gesamtgewicht der Zusammensetzung, enthalten ist.

9. Zusammensetzung nach einem der vorhergehenden Ansprüche, **dadurch gekennzeichnet, dass** der Diester der Carbonsäure in einer Konzentration im Bereich von 0,1 bis weniger als 0,5 Gew.-%, bezogen auf das Gesamtgewicht der Zusammensetzung, enthalten ist.

10. Zusammensetzung nach einem der Ansprüche 1 bis 9, **dadurch gekennzeichnet, dass** sie ferner mindestens einen für die Haare vorteilhaften Stoff enthält, der unter den pflanzlichen Ölen, tierischen Ölen, Mineralölen oder synthetischen Ölen, Wachsen, Ceramiden, Pseudoceramiden, Siliconen, kationischen Polymeren, grenzflächenaktiven Stoffen, Fettsäureestern, die von den Diestern einer Carbonsäure mit 8 bis 30 Kohlenstoffatomen und Polyethylenglycol verschieden sind, Sonnenschutzfiltern und Vitaminen ausgewählt ist.

11. Zusammensetzung nach Anspruch 10, **dadurch gekennzeichnet, dass** der für die Haare vorteilhafte Stoff in einer Konzentration vom 0,001 bis 20 Gew.-%, bezogen auf das Gesamtgewicht der Zusammensetzung, und vorzugsweise 0,01 bis 10 Gew.-% vorliegt.

12. Zusammensetzung nach einem der vorhergehenden Ansprüche, **dadurch gekennzeichnet, dass** sie ferner mindestens einen grenzflächenaktiven Stoff enthält, der unter den anionischen, nichtionischen, amphoteren oder kationischen grenzflächenaktive Stoffen und deren Gemischen ausgewählt ist.

13. Zusammensetzung nach dem vorhergehenden Anspruch, **dadurch gekennzeichnet, dass** sie ferner mindestens einen grenzflächenaktiven Stoff enthält, der unter den kationischen grenzflächenaktive Stoffen und deren Gemischen ausgewählt ist.

14. Zusammensetzung nach Anspruch 13, **dadurch gekennzeichnet, dass** der oder die kationische(n) grenzflächenaktive(n) Stoff(e) in einer Konzentration von 0,1 bis 10 Gew.-% und vorzugsweise 0,5 bis 5 Gew.-%, bezogen auf das Gesamtgewicht der Zusammensetzung, vorliegen.

15. Zusammensetzung nach einem der vorhergehenden Ansprüche, **dadurch gekennzeichnet, dass** sie als Produkt, das nach der Haarwäsche angewandt wird, als Zusammensetzung für dauerhafte Verformungen, Entkräuselungen, Färbungen oder Entfärbungen der Haare, oder als Zusammensetzung, die ausgespült wird und zwischen den beiden Schritten einer dauerhaften Verformung oder Entkräuselung aufgetragen wird, vorliegt

16. Verwendung einer Zusammensetzung, wie sie in einem der vorhergehenden Ansprüche definiert ist, für die Pflege von Keratinsubstanzen und insbesondere Haaren.

17. Verwendung einer Zusammensetzung nach einem der Ansprüche 1 bis 15 als Produkt, das nach der Haarwäsche angewandt wird.

18. Verwendung einer kosmetischen Zusammensetzung, **dadurch gekennzeichnet, dass** sie in einem kosmetisch akzeptablen Medium enthält:
- 0,1 bis 2 Gew.-% mindestens einer Stärke, bezogen auf das Gesamtgewicht der Zusammensetzung, und
- 0,1 bis 1 Gew.-% mindestens eines Diesters einer Carbonsäure mit 8 bis 30 Kohlenstoffatomen und Polyethylenglycol, bezogen auf das Gesamtgewicht der Zusammensetzung,
zum Behandeln und/oder Konditionieren der Haare.

19. Verwendung nach Anspruch 18 als Produkt, das nach der Haarwäsche angewandt wird.

20. Verfahren zur Behandlung der Haare, **dadurch gekennzeichnet, dass** es darin besteht, eine kosmetische Zusammensetzung auf die Haare aufzubringen, die in einem kosmetisch akzeptablen Medium enthält:
- 0,1 bis 2 Gew.-% mindestens einer Stärke, bezogen auf das Gesamtgewicht der Zusammensetzung, und
- 0,1 bis 1 Gew.-% mindestens eines Diesters einer Carbonsäure mit 8 bis 30 Kohlenstoffatomen und Polyethylenglycol, bezogen auf das Gesamtgewicht der Zusammensetzung,
und anschließend gegebenenfalls mit Wasser zu spülen.
